# EUROPEAN PATENT APPLICATION

(11) **EP 4 846 951 A1**
(43) Date of publication of application: **02.12.2026**
(21) Application number: 24920687.1
(22) Date of filing: 14.10.2024
(51) Int. Cl.: A61B 17/22, A61B 90/00, A61F 2/86, A61F 2/89, A61F 2/88, A61B 17/00, A61F 2/95

(54) **MECHANICAL THROMBECTOMY APPARATUS**

(30) Priority: 26.01.2024 KR 20240012201; 24.04.2024 KR 20240054804
(71) Applicant: Nventric Corporation, Seoul 08376 (KR)
(72) Inventor: NGO, Don Quy, Los Angeles, California 90065 (US); MIN, Sungwoo, Fullerton, California 92835 (US); MIN, Jiyoung, Seoul 04739 (KR)
(74) Representative: Bringer IP
(86) International application number: PCT/KR2024/015451
(87) International publication number: WO 2025/159271

(57) **Abstract**

A mechanical thrombectomy device is provided. The mechanical thrombectomy device may include a support wire, a clot arrestor, and a plurality of radiopaque markers. The clot arrestor may be connected to the support wire and may include an expandable frame. The radiopaque markers may be mounted on the expandable frame.

## Description

### [TECHNICAL FIELD]

The present disclosure relates to a thrombectomy device, and more particularly, but not limited thereto, to a mechanical thrombectomy device used for removing a thrombus from a blood vessel.

### [BACKGROUND]

Various methods exist for treating a thrombus formed at a particular blood vessel site of a body. Among them, a mechanical thrombectomy device is inserted into a blood vessel to break up or remove the thrombus within the blood vessel and is used to restore perfusion through an occluded blood vessel. Mechanical thrombectomy devices approved for such purposes include thrombus filters, thrombus aspiration devices, coil retrievers, and more recently, stent retriever devices.

A stent retriever device is primarily used to remove an acute thrombus from a stroke patient. A self-expanding stent formed of a wire mesh is deployed in a blood vessel to capture the thrombus, and the stent is then retrieved from the patient together with the thrombus by pulling a support wire of the stent retriever. In terms of usability and performance, such a stent retriever device is required to provide visibility during fluoroscopy, to capture, restrain, or retain a thrombus without damaging a blood vessel in a narrow and tortuous vascular structure, and to avoid creating dangerous situations during a procedure.

To provide visibility during fluoroscopy, conventional thrombectomy devices have provided radiopaque markers at a distal tip and on a wire or stent of the thrombectomy device. However, existing markers tend to degrade the delivery characteristics of the thrombectomy device and also provide poor marker visibility during fluoroscopic imaging.

In addition, conventional radiopaque markers require a separate support structure to be formed on a strut of the thrombectomy device in order to mount the radiopaque markers to the thrombectomy device, thereby adversely affecting the flexibility of the strut of the thrombectomy device.

### [SUMMARY]

Conventional stent retriever devices do not provide optimal visibility. Most stent retriever devices currently available are not sufficiently radiopaque in structure and therefore cannot be easily visualized during fluoroscopy in a procedure. In addition, conventional stent retriever devices have a problem in that delivery characteristics or flexibility of the stent retriever device are degraded in order to provide radiopacity.

An object of the present disclosure for solving the above-described problems is to provide a mechanical thrombectomy device having optimized visibility of a stent retriever device while not degrading delivery characteristics of the stent retriever device, by including a plurality of radiopaque markers mounted on an expandable frame.

Another object of the present disclosure for solving the above-described problems is to provide a stent retriever device capable of maintaining flexibility of the device during a procedure while providing optimal visibility during fluoroscopy by directly providing radiopaque markers having sufficient length and flexibility on an expandable frame.

However, the technical problems to be solved by the present disclosure are not limited thereto, and may be variously expanded without departing from the spirit and scope of the present disclosure.

A thrombectomy device according to one embodiment of the present disclosure for achieving the above-described objects is a mechanical thrombectomy device including: a support wire; a clot arrestor connected to the support wire and including an expandable frame; and a plurality of radiopaque markers mounted on the expandable frame.

According to one aspect, the radiopaque markers may include a plurality of end portion coil markers wound around a distal end portion of the expandable frame.

According to one aspect, the end portion coil markers may be formed of at least one material selected from the group consisting of platinum (Pt), iridium (Ir), stainless steel, gold, and tungsten.

According to one aspect, a strut forming the distal end portion of the expandable frame may include a hook-shaped portion, and the end portion coil markers may be wound so as to be caught by the hook-shaped portion.

According to one aspect, with respect to the end portion coil marker, a coil side surface of the end portion coil marker may be at least partially soldered to the strut forming the distal end portion, both ends of the end portion coil marker may be attached to the strut by an adhesive, and the hook-shaped portion may be wrapped by the adhesive.

According to one aspect, the adhesive may be tapered in a dome shape at the hook-shaped portion and be tapered in a fillet shape at the other end portion of the hook-shaped portion.

According to one aspect, the end portion coil marker, a soldering region of the end portion, and an adhesive region of the end portion may have an outer diameter equal to or less than a first threshold value required for delivery characteristics of the thrombectomy device.

According to one aspect, the radiopaque markers may include a plurality of strut coil markers wound around at least one strut forming each cell of the expandable frame.

According to one aspect, the strut coil markers may be respectively and regularly arranged on struts having the same orientation among struts forming each of the cells provided in the expandable frame.

According to one aspect, two strut coil markers may be arranged in one cell, and the two strut coil markers may be arranged to be spaced apart from each other in a longitudinal direction of the expandable frame about a transverse division axis of the cell.

According to one aspect, the strut coil markers may be arranged to be spaced apart from one another at predetermined intervals between the respective cells.

According to one aspect, the strut coil markers may be arranged in an oblique direction with respect to a longitudinal axis of the clot arrestor.

According to one aspect, the strut coil markers may be arranged to be spaced apart from one another in a longitudinal direction of the expandable frame.

According to one aspect, the plurality of strut coil markers may be arranged at positions different from one another in a radial direction of the expandable frame.

According to one aspect, with respect to the strut coil marker, a coil side surface of the strut coil marker may be at least partially soldered to the strut, and both ends of the strut coil marker may be attached to the strut by an adhesive.

According to one aspect, the strut may have flexibility, and the soldering may have a longitudinal length that maintains the flexibility of the strut even when the soldering is attached to the strut coil marker.

According to one aspect, the adhesive may be tapered in a fillet shape.

According to one aspect, the strut coil marker and a soldering region of the strut may have an outer diameter equal to or less than a second threshold value required for delivery characteristics of the thrombectomy device.

According to one aspect, the strut coil marker may be formed of at least one material selected from the group consisting of platinum (Pt), iridium (Ir), stainless steel, gold, and tungsten.

The disclosed technology may provide the following effects. However, this does not mean that a particular embodiment must include all of the following effects or only the following effects. Accordingly, the scope of the disclosed technology should not be construed as being limited thereby.

According to the mechanical thrombectomy device of one embodiment of the present disclosure described above, the radiopaque markers are mounted on the struts of the expandable frame without providing separate support structures, branches, or the like, thereby making it possible to provide a mechanical thrombectomy device having optimized visibility of the stent retriever device without degrading delivery characteristics of the device during delivery of the mechanical thrombectomy device to a treatment site and retrieval of the mechanical thrombectomy device from the treatment site.

In addition, the mechanical thrombectomy device according to one embodiment of the present disclosure described above directly provides the radiopaque markers having sufficiently thin, long, and flexible characteristics on the struts of the expandable frame, thereby not only providing optimal visibility throughout the thrombectomy device during fluoroscopy but also maintaining flexibility of the device during a procedure, thus providing a mechanical thrombectomy device having excellent usability.

### [BRIEF DESCRIPTION OF THE DRAWINGS]

FIG. 1 is a plan view of a mechanical thrombectomy device according to one embodiment of the present disclosure.
FIG. 2 is a two-dimensional plan view of an expandable frame illustrating an end portion coil marker according to one embodiment of the present disclosure.
FIG. 3 is an enlarged view of the end portion coil marker according to one embodiment of the present disclosure.
FIG. 4 is an enlarged view of a distal end portion strut of the mechanical thrombectomy device according to one embodiment of the present disclosure.
FIG. 5 is a fluoroscopic view of the end portion coil marker according to one embodiment of the present disclosure.
FIG. 6 is a two-dimensional plan view of an expandable frame illustrating a strut coil marker according to one embodiment of the present disclosure.
FIG. 7 is an enlarged view of the strut coil marker according to one embodiment of the present disclosure.
FIG. 8 is a view illustrating a photograph of the strut coil marker according to one embodiment of the present disclosure.
FIG. 9 is a fluoroscopic view of the strut coil marker according to one embodiment of the present disclosure.
FIG. 10 is a two-dimensional plan view of an expandable frame illustrating another arrangement of the strut coil marker according to one embodiment of the present disclosure.
FIG. 11 is a partial two-dimensional plan view of an expandable frame illustrating still another arrangement of the strut coil marker according to one embodiment of the present disclosure.
FIG. 12 is a partial two-dimensional plan view of an expandable frame illustrating yet another arrangement of the strut coil marker according to one embodiment of the present disclosure.
FIG. 13 is a schematic view for explaining yet another arrangement of the strut coil marker according to one embodiment of the present disclosure.
FIG. 14 is a two-dimensional plan view of an expandable frame illustrating yet another arrangement of the strut coil marker according to one embodiment of the present disclosure.
FIG. 15 is a schematic view illustrating an arrangement of the strut coil markers as viewed from a distal end of a clot arrestor according to one embodiment of the present disclosure.

### [DETAILED DESCRIPTION]

The present disclosure may be modified in various ways and may have various embodiments, and specific embodiments will be illustrated in the drawings and described in detail.

However, this is not intended to limit the present disclosure to particular embodiments, and it should be understood that all modifications, equivalents, or substitutes falling within the spirit and technical scope of the present disclosure are included therein.

Terms such as first and second may be used to describe various elements, but the elements should not be limited by these terms. These terms are used only for the purpose of distinguishing one element from another element. For example, a first element may be referred to as a second element without departing from the scope of the present disclosure, and similarly, a second element may be referred to as a first element. The term "and/or" includes any combination of a plurality of related listed items or any one of the plurality of related listed items.

When an element is referred to as being "connected" or "coupled" to another element, it should be understood that it may be directly connected or coupled to the other element, or intervening elements may be present therebetween. In contrast, when an element is referred to as being "directly connected" or "directly coupled" to another element, it should be understood that no intervening element is present therebetween.

The terminology used herein is for the purpose of describing particular embodiments only and is not intended to limit the present disclosure. Singular forms include plural forms unless the context clearly indicates otherwise. As used herein, the terms such as "include" and "have" are intended to specify the presence of stated features, numbers, steps, operations, elements, components, parts, or combinations thereof, and should not be construed as precluding the presence or addition of one or more other features, numbers, steps, operations, elements, components, parts, or combinations thereof.

Unless otherwise defined, all terms used herein, including technical and scientific terms, have the same meanings as commonly understood by a person having ordinary skill in the art to which the present disclosure pertains. Terms defined in commonly used dictionaries should be interpreted as having meanings consistent with their meanings in the context of the relevant art and should not be interpreted in an idealized or overly formal sense unless expressly defined herein.

Hereinafter, preferred embodiments will be described in greater detail with reference to the accompanying drawings. In describing the present disclosure, the same reference numerals are used to designate the same elements throughout the drawings in order to facilitate overall understanding of the present disclosure, and redundant descriptions of the same elements will be omitted.

As discussed above, conventional stent retrievers have mounted separate branches on struts in order to install radiopaque markers. Accordingly, when it is desired to make the radiopaque markers sufficiently visible, the number or length of the branches had to be increased. As a result, although visibility is improved, the radial profile of the stent retriever increases, thereby causing a problem in that usability during delivery of the device in a procedure is significantly degraded. Accordingly, it has not been practically possible to provide sufficiently visible radiopaque markers. In addition, conventional stent retrievers having separate support structures for radiopaque markers have a problem in that flexibility of an expandable frame of the stent retriever is reduced when the radiopaque markers are mounted, which becomes a factor that impairs delivery performance of the device during a procedure.

A mechanical thrombectomy device according to one embodiment of the present disclosure is intended to solve the above-described problems and can provide a plurality of radiopaque markers that are directly mounted on an expandable frame and are sufficiently visible and flexible. In addition, by effectively arranging the plurality of radiopaque markers so as not to affect a radial profile of the mechanical thrombectomy device, a mechanical thrombectomy device having excellent usability may be provided.

Hereinafter, a mechanical thrombectomy device according to one embodiment will be described in more detail with reference to the drawings.

Hereinafter, in the present disclosure, a "clot arrestor" may refer, for example, to a portion of a general stent retriever including a stent. However, the clot arrestor is not limited to a conventional stent retriever and should be understood as including all components required to restore perfusion by removing coagulated blood from a blood vessel.

Further, hereinafter, in the present disclosure, an "expandable frame" refers to a frame that is initially maintained in a compressed state and may be expanded after being moved to an appropriate location, and it should be understood that any form known in the art may be applied thereto.

In addition, the use of relative terms throughout the Description of the Invention of the present specification may indicate relative positions or directions. For example, "distal" may indicate a first direction along a longitudinal axis of a support wire or a clot arrestor. Likewise, "proximal" may indicate a second direction opposite to the first direction. For example, a longitudinal direction of the thrombectomy device may indicate a direction along a longitudinal central axis of the clot arrestor, a transverse direction may indicate a direction crossing the longitudinal central axis of the clot arrestor, and a longitudinal axis may indicate the longitudinal central axis of the clot arrestor. However, these terms are provided to establish relative references and are not intended to limit the use or orientation of the mechanical thrombectomy device to specific configurations described in the various embodiments below.

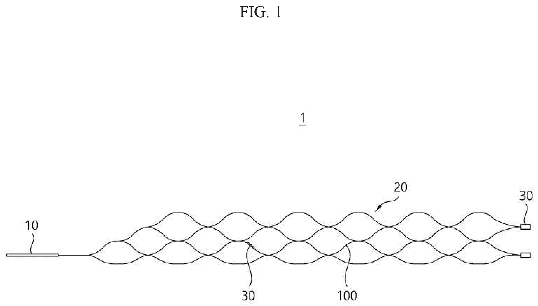

FIG. 1 is a plan view of a mechanical thrombectomy device according to one embodiment of the present disclosure. As illustrated in FIG. 1, a mechanical thrombectomy device 1 according to one embodiment may include, for example, a support wire 10, a clot arrestor 20, and radiopaque markers 30.

The mechanical thrombectomy device 1 is, for example, an endovascular tool that may be used to treat acute ischemic stroke. The mechanical thrombectomy device 1 includes a proximal control region that is used by an operator to advance, retrieve, and rotate a distal operating region of the device. More specifically, the mechanical thrombectomy device 1 includes a support wire 10 that may be pushed by the operator to advance the distal operating region, pulled by the operator to retrieve the distal operating region, or rotated by the operator to rotate the distal operating region. In one embodiment, the support wire 10 may be a flexible elongated wire formed of a resilient material such as stainless steel or a super-elastic nickel titanium alloy.

The mechanical thrombectomy device 1 may include the clot arrestor 20 that may be advanced through a microcatheter and deployed from the microcatheter to a target tissue. When deployed within the target tissue, the clot arrestor 20 may capture, entrap, restrain, or mechanically integrate with a thrombus. The restrained thrombus may then be retrieved from a patient by pulling the support wire 10 to retrieve the clot arrestor 20 and the thrombus from a vascular structure.

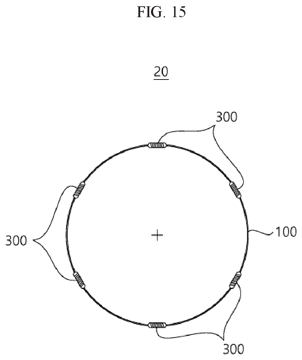

The clot arrestor 20 may be sized and shaped to provide respective degrees of thrombus restraint, thrombus capture, flexibility, or any other performance characteristic. The clot arrestor 20 may be connected to a distal end of the support wire 10 and may include an expandable frame 100. The expandable frame 100 may have a generally elongated cylindrical frame structure capable of capturing and restraining a thrombus within a cylindrical interior thereof, and may be manufactured from a shape memory alloy material including a nickel titanium alloy having flexibility, shape memory, and contraction characteristics. The size and shape of each cell of the expandable frame 100 may be determined according to the above-described performance characteristics of the thrombectomy device. The clot arrestor 20 may be formed by laser-cutting a cylindrical tube into a three-dimensional expandable structure, or may be formed by machining, chemical machining, electromechanical machining, electrical discharge machining, or various methods known in the art. Accordingly, the expandable frame 100 of the clot arrestor 20 may have a circular cross-sectional profile, as illustrated in FIG. 15 to be described later.

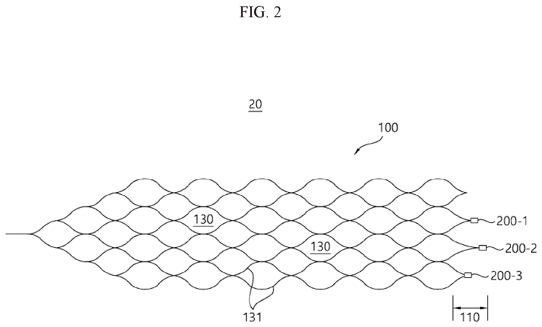

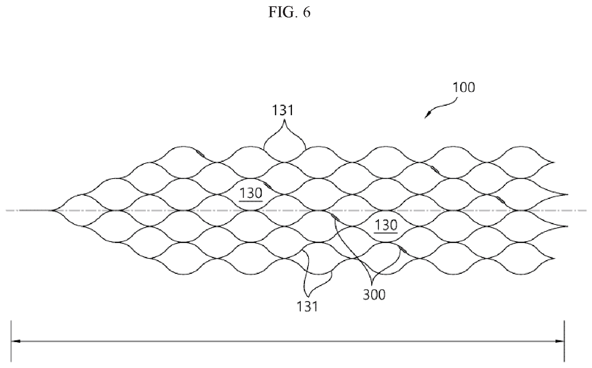

A plurality of radiopaque markers 30 may be mounted on the expandable frame 100. The radiopaque marker 30 may include an end portion coil marker 200 and a strut coil marker 300, which will be described in detail below with reference to FIGS. 2 and 6. The radiopaque marker 30 may increase visibility of the device and provide a clue that allows an operator to recognize a position of a thrombus with respect to the clot arrestor. The radiopaque marker 30 may include a radiopaque marker mounted by various methods including a radiopaque band, a wire coil, plating, welding, coating, or the like, which are commonly used in the art. Alternatively, a radiopaque material may be plated on the expandable frame 100. Alternatively, the radiopaque marker 30 may be formed as bands crimped onto the expandable frame 100. In one aspect, the radiopaque marker 30 may include coils formed of a radiopaque wire and may be wound or wrapped around the expandable frame 100. In one aspect, the radiopaque marker 30 may have an atraumatic surface that does not damage a blood vessel wall during use.

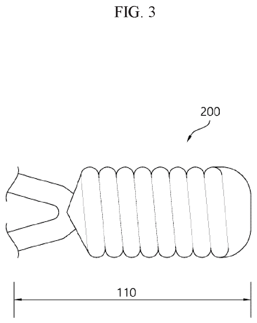

FIG. 2 is a two-dimensional plan view of the expandable frame 100 illustrating the end portion coil markers 200 according to one embodiment of the present disclosure, and FIG. 3 is an enlarged view of the end portion coil marker illustrated in FIG. 2. The expandable frame 100 will first be described with reference to FIG. 2. The expandable frame 100 may be formed by a plurality of struts 131 forming respective cells 130. Although each cell 130 is illustrated as having a generally sinusoidal pattern, the pattern of the cell 130 is not limited thereto, and any form according to performance characteristics of the clot arrestor may be possible.

Referring to FIGS. 2 and 3, a plurality of end portion coil markers 200 may be mounted on a distal end portion 110 of the expandable frame 100. More specifically, the end portion coil markers 200 may be wound around struts forming the distal end portion 110 of the expandable frame 100. The cells 130 of the expandable frame 100 forming the distal end portion 110 have different shapes, and ends of the respective cells around which the end portion coil markers 200 are wound are offset from one another in a longitudinal direction of the expandable frame 100. Accordingly, the plurality of end portion coil markers 200-1, 200-2, and 200-3 may also be wound around the expandable frame 100 while being offset from one another in the longitudinal direction of the expandable frame 100.

The end portion coil marker 200 may be formed of at least one radiopaque material selected from the group consisting of platinum (Pt), iridium (Ir), stainless steel, gold, and tungsten. Accordingly, the end portion coil marker 200 may provide visibility of the distal end portion 110 of the thrombectomy device 1.

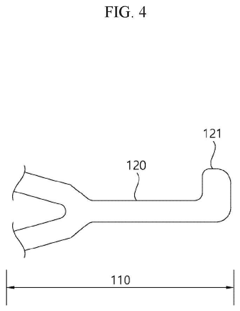

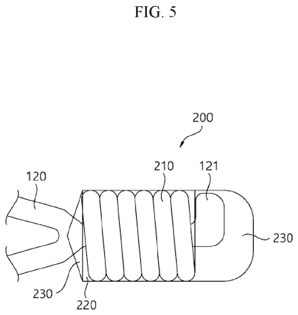

Reference is made to FIGS. 4 and 5 to describe the structure of the end portion coil marker 200 in greater detail. FIG. 4 is an enlarged view of a strut 120 of the distal end portion 110 of the mechanical thrombectomy device 1 according to one embodiment of the present disclosure, and FIG. 5 is a fluoroscopic view of the end portion coil marker 200 according to one embodiment of the present disclosure.

Referring to FIGS. 3 to 5, in the distal end portion 110 of the expandable frame, a linear strut 120 may extend in the longitudinal direction of the expandable frame 100 from a distalmost cell of the expandable frame 100. The strut 120 forming the distal end portion 110 of the expandable frame 100 may have a hook-shaped portion 121 at a distal end thereof, and the hook-shaped portion 121 may be bent and extend in a transverse direction of the expandable frame 100 so that the end portion coil marker 200 may be caught thereon, as illustrated in FIG. 5.

More specifically, referring to FIG. 5, the end portion coil marker 200 may include a coil 210, a region of the soldering 220, and a region of the adhesive 230. A side surface of the coil 210 of the end portion coil marker 200 is wound around the strut 120 forming the distal end portion 110, and the side surface of the coil 210 may be fixed to the strut 120 by being at least partially soldered 220 to the strut 120. In addition, both ends of the end portion coil marker 200 may be attached to the strut 120 by an adhesive 230. In this case, the adhesive 230 may be applied in a form that wraps the hook-shaped portion 121.

The adhesive 230 may be attached to both ends of the coil 210 and then tapered, and the adhesive 230 formed to wrap the hook-shaped portion 121 is tapered in a dome shape so as to sufficiently cover the hook-shaped portion 121 and form a smooth curve. In addition, the adhesive 230 applied to the other end portion of the hook-shaped portion 121 may be tapered into a fillet shape. Due to such tapering, edges or stepped portions of the strut 120 and the hook-shaped portion 121 to which the end portion coil marker 200 is attached may be smoothly wrapped, thereby reducing frictional force when the mechanical thrombectomy device 1 moves within a blood vessel and providing an atraumatic surface capable of preventing damage to a blood vessel wall.

The coil 210, the region of the soldering 220, and the region of the adhesive 230 of the end portion coil marker 200 described above should have an outer diameter equal to or less than a first threshold value required for delivery characteristics of the mechanical thrombectomy device 1 of the present disclosure. The mechanical thrombectomy device 1 of the present disclosure may have delivery characteristics respectively required for details such as flexibility, compressibility, a delivery system, and accuracy when the device is delivered along a curve of a blood vessel to capture and retrieve an occluding thrombus in the blood vessel and improve blood flow. Such delivery characteristics may be determined according to details required during a procedure.

As described above, when the plurality of end portion coil markers 200 are arranged on the distal end portion 110 of the expandable frame 100 while being offset from one another, the coil 210, the region of the soldering 220, and the region of the adhesive 230 of the coil marker 200 may have an outer diameter equal to or less than the first threshold value that satisfies the respective delivery characteristics required for the mechanical thrombectomy device 1 of the present disclosure in such an arrangement relationship. Accordingly, the mechanical thrombectomy device 1 can be used safely and accurately during a procedure.

A range of the outer diameter equal to or less than the first threshold value of the side surface of the coil 210, the region of the soldering 220, and the region of the adhesive 230 of the coil marker 200 may be, for example, 0.5 to 0.3 mm, and preferably 0.33 ± 0.02 mm. In addition, a length of the soldering may have a specific soldering length required so as not to exceed a required amount of soldering. A range of the length of such soldering may be, for example, 0.5 to 0.4 mm, and preferably 0.40 ± 0.05 mm. In addition, a length of the adhesive may have a length required to form a dome shape or a fillet, and may have a range of less than 0.2 mm.

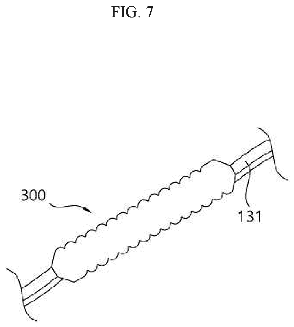

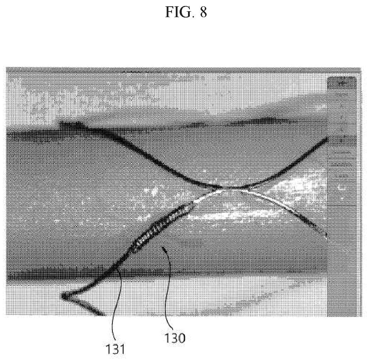

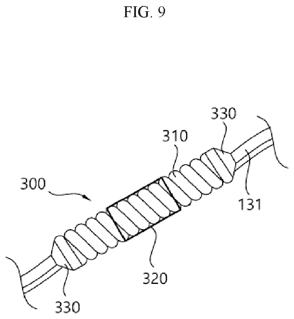

FIG. 6 is a two-dimensional plan view of an expandable frame illustrating a strut coil marker according to one embodiment of the present disclosure, FIG. 7 is an enlarged view of the strut coil marker according to one embodiment of the present disclosure, FIG. 8 is a view illustrating a photograph of the strut coil marker according to one embodiment of the present disclosure, and FIG. 9 is a fluoroscopic view of the strut coil marker according to one embodiment of the present disclosure. Hereinafter, the strut coil marker 300 according to an aspect of the present disclosure will be described in greater detail with reference to FIGS. 6 to 9.

As illustrated in FIGS. 6 to 9, a radiopaque strut coil marker 300 according to one embodiment may be a plurality of coil markers that individually surround and are wound around at least one strut 131 forming each cell 130 of the expandable frame 100, and may provide radiopacity without impairing a delivery profile of the mechanical thrombectomy device 1. The strut coil marker 300 may be formed of at least one radiopaque material selected from the group consisting of platinum (Pt), iridium (Ir), stainless steel, gold, and tungsten. Accordingly, the strut coil marker 300 may provide visibility of an operating region of the expandable frame 100 of the thrombectomy device 1.

In one embodiment of the present disclosure, the strut coil marker 300 may include a coil 310, a region of the soldering 320, and a region of the adhesive 330, as illustrated in detail in FIG. 9. A side surface of the coil 310 of the strut coil marker 300 may be fixed to the strut 131 forming each cell 130 by being at least partially soldered 320 to the strut 131.

The strut 131 on which the strut coil marker 300 is mounted has flexibility, and the soldering 320 may be formed to have a length that maintains flexibility without affecting the flexibility of the strut 131 even when the strut coil marker 300 is attached to the strut 131 by the soldering 320.

Alternatively, in order to maintain flexibility, a material of the soldering 320 may be selected as an alloy material having flexibility, or the soldering 320 may be formed to have a short length so as not to affect flexibility of the coil 310 and the strut 131.

Alternatively, a portion of the strut 131 on which the strut coil marker 300 is mounted may be tapered or ground so that a diameter of the strut coil marker 300 may be maintained small even when the strut coil marker 300 is mounted. Accordingly, a packing ratio of the mechanical thrombectomy device 1 can be improved (that is, a smaller unexpanded profile can be achieved), thereby facilitating improved delivery of the device through a microcatheter.

In addition, both ends of the strut coil marker 300 may be attached to the strut 131 by the adhesive 330. The adhesive 330 may be attached to an end of the coil 310 and then tapered in a fillet shape. Due to such tapering, edges of the strut 131 to which the strut coil marker 300 is attached or stepped portions of the strut coil marker 300 may be smoothly wrapped, thereby reducing frictional force when the mechanical thrombectomy device 1 moves within a blood vessel and providing an atraumatic surface capable of preventing damage to a blood vessel wall during use.

Meanwhile, the side surface of the coil 310 and the region of the soldering 320 of the strut coil marker 300 described above may have an outer diameter equal to or less than a second threshold value required for delivery characteristics of the mechanical thrombectomy device 1 of the present disclosure. The mechanical thrombectomy device 1 of the present disclosure may have delivery characteristics respectively required for details such as flexibility, compressibility, a delivery system, and accuracy when the device is delivered along a curve of a blood vessel to capture and retrieve an occluding thrombus in the blood vessel and improve blood flow, and such delivery characteristics may be determined according to details required during a procedure.

As described above, when the plurality of strut coil markers 300 are directly wound and arranged on the respective struts 131 of the expandable frame 100, the strut coil markers 300 have an outer diameter equal to or less than the second threshold value that satisfies the respective delivery characteristics required for the mechanical thrombectomy device 1 of the present disclosure. Accordingly, the mechanical thrombectomy device 1 can be used safely and accurately during a procedure. A range of the outer diameter equal to or less than the second threshold value, which may not affect the delivery profile of the coil 310 and the soldering 320 of the strut coil marker 300, may be, for example, 0.2 to 0.15 mm, and preferably 0.18 ± 0.01 mm.

In addition, the soldering 320 should not exceed a required amount of soldering and should have a soldering length formed to a length that does not affect flexibility of the coil 310 and the strut 131. A range of the length of such soldering may be, for example, 0.5 to 0.15 mm, and preferably 0.30 ± 0.10 mm. A length of the coil 310 should also have a coil length range that may not overlap the coil 310 of another strut coil marker 300 and adversely affect the delivery profile. A range of such a coil length may be, for example, 1.5 to 0.5 mm, and preferably 1.0 ± 0.1 mm. In addition, a length of the adhesive 330 forming fillets at both ends of the coil 310 may have a range of less than 0.2 mm, similarly to the end portion coil marker 200.

Alternatively, for example, an aspect ratio of a length of the coil 310 to a diameter of the coil 310 of the strut coil marker 300 may be in a range of 5 to 6. Preferably, for example, the aspect ratio of the length of the coil 310 to the diameter of the coil 310 of the strut coil marker 300 may be in a range of 5.2 to 5.8. Since the strut coil marker 300 having such an aspect ratio has a length relatively at least five times greater than its diameter, more radiopaque material may be included in the strut coil marker 300, and accordingly, better visibility may be provided in the operating region when the mechanical thrombectomy device 1 is used.

Since the above-described ranges for the outer diameters of the coil 310 and the soldering 320 of the strut coil marker 300 and for the lengths of the coil 310, the soldering 320, and the adhesive 330 are ranges that do not affect delivery characteristics when the mechanical thrombectomy device 1 is used, it is possible to provide the mechanical thrombectomy device 1 having excellent usability. In addition, since the length of the coil 310 is relatively longer than radiopaque markers of competing products, visibility of the marker during use can be greatly improved, and accordingly, accuracy when using the mechanical thrombectomy device 1 may also be improved. In addition, since the strut coil marker 300 is directly wound around the strut 131 and the soldering 320 is partially applied to the side surface of the coil 310 as illustrated in FIGS. 7 to 9, it is possible to provide the mechanical thrombectomy device 1 having excellent delivery characteristics during use while maintaining flexibility of the coil 310 and the strut 131.

Referring again to FIG. 6, an arrangement of the strut coil markers 300 according to one embodiment of the present disclosure will be described. As illustrated in FIG. 6, in the two-dimensional plan view of the expandable frame 100, the plurality of strut coil markers 300 may be regularly arranged on struts 131 having the same orientation among the struts 131 forming the respective cells 130. In the embodiment of FIG. 6, the respective strut coil markers 300 may be arranged so as not to overlap one another in the longitudinal direction of the expandable frame 100 indicated by an arrow. In addition, the respective strut coil markers 300 may also be arranged so as not to overlap one another in the transverse direction of the expandable frame 100. Since such an arrangement provides a smaller, unexpanded profile of the thrombectomy device, it may help improve delivery of the thrombectomy device through a microcatheter, thereby providing a thrombectomy device having excellent usability and accuracy. Accordingly, a rule for arranging the strut coil markers 300 may be applied in any possible combination within a range in which the strut coil markers 300 do not overlap one another in the longitudinal direction and the transverse direction of the expandable frame 100.

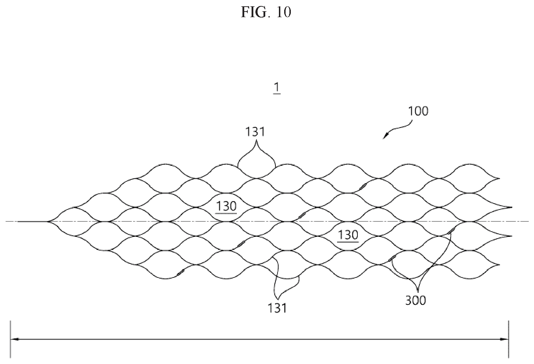

FIG. 10 is a two-dimensional plan view of an expandable frame illustrating another arrangement of the strut coil markers according to one embodiment of the present disclosure. In the embodiment of FIG. 10, similarly to FIG. 6, the respective strut coil markers 300 are regularly arranged on struts 131 having the same orientation among the struts 131 forming the respective cells 130 provided in the expandable frame 100. However, the orientation of the struts 131 is different from that illustrated in FIG. 6. Since the same effects as those of the arrangement of the strut coil markers 300 illustrated in FIG. 6 may also be applied to the arrangement of the strut coil markers 300 illustrated in FIG. 10, descriptions corresponding to FIG. 6 will be omitted.

In the embodiments illustrated in FIGS. 6 and 10, all of the strut coil markers 300 may be arranged in an oblique direction with respect to the longitudinal axis of the clot arrestor in the longitudinal direction of the expandable frame 100 indicated by an arrow. Such an oblique arrangement allows the strut coil markers 300 to be visualized as a whole in the longitudinal direction of the device without increasing the radial profile of the device during compression, compared to a case in which the strut coil markers 300 are arranged in a straight line in the longitudinal direction when the thrombectomy device 1 is compressed in the longitudinal direction.

In addition, in the embodiments illustrated in FIGS. 6 and 10, the strut coil markers 300 may be arranged to be spaced apart from one another at predetermined intervals between the respective cells 130. Referring to FIGS. 6 and 10, when each strut coil marker 300 is viewed in an oblique direction with respect to the longitudinal direction of the expandable frame 100, a cell 130 in which the strut coil marker 300 is not arranged exists between cells 130 in which the strut coil marker 300 is arranged. The number or arrangement of the cells 130 in which the strut coil markers 300 are not arranged may be determined according to what is required for the performance characteristics of the device. Since the strut coil markers 300 are spaced apart from one another at uniform intervals in such an arrangement, uniform visibility may be provided over the longitudinal length of the expandable frame 100.

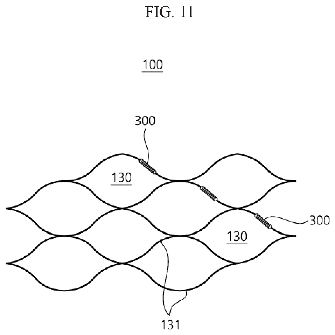

FIG. 11 is a partial two-dimensional plan view of the expandable frame 100 illustrating still another arrangement of the strut coil markers 300 according to one embodiment of the present disclosure. In the embodiment illustrated in FIG. 11, the strut coil markers 300 may be arranged on struts 131 having the same orientation, which form the respective cells 130, in an oblique direction with respect to the longitudinal axis of the expandable frame 100. In this case, the strut coil markers 300 may be continuously arranged in the respective cells 130 without a cell 130 in which the strut coil marker 300 is not arranged being present between the respective cells 130. Such an arrangement may have an advantage in that the radiopaque strut coil markers 300 can be visualized as appearing longer in the longitudinal direction of the expandable frame 100 than in the arrangements of the strut coil markers 300 illustrated in FIGS. 6 and 10.

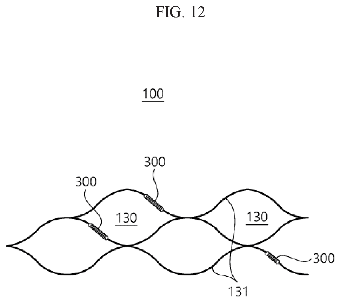

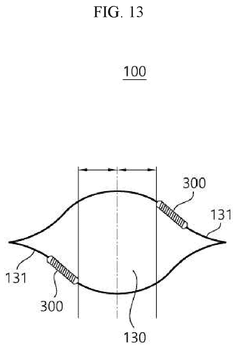

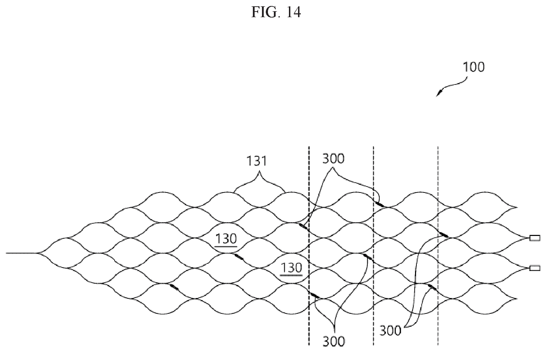

FIG. 12 is a partial two-dimensional plan view of the expandable frame 100 illustrating yet another arrangement of the strut coil markers 300 according to one embodiment of the present disclosure, FIG. 13 is a schematic view for explaining yet another arrangement of the strut coil markers 300 according to one embodiment of the present disclosure, and FIG. 14 is a two-dimensional plan view of an expandable frame illustrating yet another arrangement of the strut coil markers 300 according to one embodiment of the present disclosure.

In the embodiment of FIG. 12, two strut coil markers 300 may be arranged in one cell 130 of the expandable frame 100. When two strut coil markers 300 are arranged in one cell 130, the strut coil markers 300 may be arranged opposite to each other on struts 131 having the same orientation, and may be arranged to be spaced apart from one another at predetermined intervals between the respective cells 130 according to a certain rule.

In one aspect, as illustrated in FIG. 12, two strut coil markers 300 arranged in one cell 130 at an upper portion of the expandable frame 100 and one strut coil marker 300 arranged at a lower portion of the expandable frame 100 may have different lengths. Such an arrangement of the strut coil markers 300 having different lengths may be used to highlight and indicate a specific portion of the thrombectomy device 1. That is, such an arrangement may be used to more easily visualize a desired position by arranging relatively longer strut coil markers 300 at a specific position of the clot arrestor 20 so that a target action point of the clot arrestor 20 can be more clearly identified as needed.

More specifically, referring to FIG. 13, two or more strut coil markers 300 may be arranged in one cell 130. For example, two strut coil markers 300 may be arranged on opposing struts 131 while being spaced apart from each other in the longitudinal direction of the expandable frame 100 with respect to a transverse division axis (illustrated by a dotted line) of the cell 130.

In the embodiment illustrated in FIG. 13, the two strut coil markers 300 arranged in one cell 130 may be arranged at positions that do not overlap each other in the longitudinal direction of the expandable frame 100. The two strut coil markers 300 may be arranged to be spaced apart from each other by the same distance in the longitudinal direction of the expandable frame 100, as respectively indicated by arrows, with respect to the transverse division axis of the expandable frame 100. Such a spacing distance may vary depending on the length of the strut coil marker 300.

FIG. 14 is a two-dimensional plan view of the expandable frame 100 illustrating yet another arrangement of the strut coil markers 300 according to one embodiment of the present disclosure. In the embodiment of FIG. 14, the strut coil markers 300 may be regularly arranged on struts 131 having the same orientation among the struts 131 forming the respective cells 130 with respect to a transverse division axis of the expandable frame 100. The strut coil markers 300 illustrated in FIG. 14 may have an arrangement in which they are spaced apart from one another in the longitudinal direction of the expandable frame 100, similarly to the embodiments of FIGS. 6 and 10. However, the arrangement differs in that the strut coil markers 300 are arranged to be spaced apart from one another in the transverse direction in the cells 130 on the same line with respect to the transverse direction of the expandable frame 100 indicated by dotted lines.

When the strut coil markers 300 are respectively arranged on struts 131 having the same orientation in the cells 130 located on the same line in the transverse direction, the strut coil markers 300 may be arranged to be spaced apart from each other in the longitudinal direction of the expandable frame 100 while being spaced apart on both sides with respect to transverse division axes illustrated by dotted lines in FIG. 14. Accordingly, the strut coil markers 300 may be arranged closer to one another in the longitudinal direction of the expandable frame 100 than when two strut coil markers 300 are respectively arranged on opposing struts 131 in one cell 130 as illustrated in FIG. 13. However, even in such an arrangement, the strut coil markers 300 may be spaced apart from one another without overlapping one another in the longitudinal direction of the expandable frame 100.

When viewed in the longitudinal direction of the expandable frame 100 during use, the strut coil markers 300 arranged as illustrated in FIG. 14 may appear combined and longer, like one strut coil marker 300, rather than two strut coil markers 300 arranged in the cells 130 on the same line in the transverse direction, and this may also have an effect of increasing visibility of the radiopaque markers.

FIG. 15 is a schematic view illustrating an arrangement of the strut coil markers 300 as viewed from a distal end of the clot arrestor 20 according to one embodiment of the present disclosure. An arrangement of the strut coil markers 300 in the radial direction according to one embodiment of the present disclosure will be described with reference to FIG. 15. Although the plurality of strut coil markers 300 are illustrated in two-dimensional plan views in FIGS. 6 to 14 for purposes of description, during actual use, they may be distributed while being radially spaced apart from one another over the circumferential direction of the generally cylindrical expandable frame 100. The strut coil markers 300 may be arranged at different positions in the radial direction of the expandable frame 100, for example, as illustrated in FIG. 15. Preferably, the strut coil markers 300 may be arranged at substantially equal intervals in the radial direction when viewed from an end of each expandable frame 100 as illustrated in FIG. 15. However, such intervals do not necessarily have to be equal, and the strut coil markers 300 may be arranged in a manner suitable for the delivery characteristics of the mechanical thrombectomy device 1 without increasing the radial profile of the mechanical thrombectomy device 1.

Due to the above-described arrangement in which the respective strut coil markers 300 of the present disclosure are spaced apart in the longitudinal direction and the transverse direction of the expandable frame 100, the strut coil markers 300 may not overlap one another, and the radial profile of the mechanical thrombectomy device 1 may not increase. In addition, since the mechanical thrombectomy device 1 according to one embodiment of the present disclosure has the configuration and arrangement of the radiopaque markers 30 as described above, visibility of the radiopaque markers 30 may be remarkably improved compared to conventional stent retriever devices, and a smaller, unexpanded profile of the mechanical thrombectomy device 1 may be provided, thereby providing improved delivery performance of the mechanical thrombectomy device 1 through a microcatheter.

In addition, for example, as illustrated in FIG. 6, the strut coil markers 300 are arranged in an oblique direction with respect to the longitudinal axis of the clot arrestor 20 and are arranged in a spiral form in the radial direction, thereby expanding a radial arrangement region. Accordingly, fluoroscopy may be performed regardless of the radial orientation of the clot arrestor 20 with respect to a fluoroscopic apparatus.

Although the present disclosure has been described above with reference to the drawings and embodiments, this does not mean that the scope of protection is limited by the drawings or embodiments. A person skilled in the art will understand that the present disclosure may be variously modified and changed without departing from the spirit and scope of the present disclosure described in the following claims.

The present disclosure described above is not limited by the above-described embodiments and the accompanying drawings, and it will be apparent to a person having ordinary skill in the art to which the present disclosure pertains that various substitutions, modifications, and changes may be made without departing from the technical spirit of the present disclosure.

Combinations of the above-described embodiments are not limited to the above-described embodiments, and various forms of combinations may be provided according to implementation and/or need, in addition to the above-described embodiments.

The above-described embodiments include examples of various aspects. Although all possible combinations for representing the various aspects cannot be described, a person having ordinary skill in the art will recognize that other combinations are possible. Accordingly, the present disclosure should be understood as including all other substitutions, modifications, and changes falling within the scope of the following claims.

### [DESCRIPTION OF REFERENCE NUMERALS]

1: mechanical thrombectomy device
10: support wire
20: clot arrestor
30: radiopaque marker
100: expandable frame
110: distal end portion
120: strut (forming distal end portion)
121: hook-shaped portion
130: cell
131: strut
200: end portion coil marker
210: coil side surface
220: soldering
230: adhesive
300: strut coil marker
310: coil side surface
320: soldering
330: adhesive

## Claims

1. A mechanical thrombectomy device comprising:
a support wire;
a clot arrestor connected to the support wire and including an expandable frame; and
a plurality of radiopaque markers mounted on the expandable frame.

2. The mechanical thrombectomy device of claim 1, wherein the radiopaque markers include a plurality of end portion coil markers wound around a distal end portion of the expandable frame.

3. The mechanical thrombectomy device of claim 2, wherein the end portion coil markers are formed of at least one material selected from the group consisting of platinum (Pt), iridium (Ir), stainless steel, gold, and tungsten.

4. The mechanical thrombectomy device of claim 2, wherein a strut forming the distal end portion of the expandable frame includes a hook-shaped portion, and
the end portion coil markers are wound so as to be caught by the hook-shaped portion.

5. The mechanical thrombectomy device of claim 4, wherein, with respect to the end portion coil marker,
a coil side surface of the end portion coil marker is at least partially soldered to the strut forming the distal end portion,
both ends of the end portion coil marker are attached to the strut by an adhesive, and
the hook-shaped portion is wrapped by the adhesive.

6. The mechanical thrombectomy device of claim 5, wherein the adhesive is tapered in a dome shape at the hook-shaped portion and is tapered in a fillet shape at the other end portion of the hook-shaped portion.

7. The mechanical thrombectomy device of claim 6, wherein the end portion coil marker, a soldering region of the end portion, and an adhesive region of the end portion have an outer diameter equal to or less than a first threshold value required for delivery characteristics of the thrombectomy device.

8. The mechanical thrombectomy device of claim 1, wherein the radiopaque markers include a plurality of strut coil markers wound around at least one strut forming each cell of the expandable frame.

9. The mechanical thrombectomy device of claim 8, wherein the strut coil markers are respectively and regularly arranged on struts having the same orientation among struts forming each of the cells provided in the expandable frame.

10. The mechanical thrombectomy device of claim 9, wherein two strut coil markers are arranged in one cell, and the two strut coil markers are arranged to be spaced apart from each other in a longitudinal direction of the expandable frame about a transverse division axis of the cell.

11. The mechanical thrombectomy device of claim 9, wherein the strut coil markers are arranged to be spaced apart from one another at predetermined intervals between the respective cells.

12. The mechanical thrombectomy device of claim 9, wherein the strut coil markers are arranged in an oblique direction with respect to a longitudinal axis of the clot arrestor.

13. The mechanical thrombectomy device of claim 12, wherein the strut coil markers are arranged to be spaced apart from one another in a longitudinal direction of the expandable frame.

14. The mechanical thrombectomy device of claim 12, wherein the plurality of strut coil markers are arranged at positions different from one another in a radial direction of the expandable frame.

15. The mechanical thrombectomy device of claim 8, wherein, with respect to the strut coil marker,
a coil side surface of the strut coil marker is at least partially soldered to the strut, and
both ends of the strut coil marker are attached to the strut by an adhesive.

16. The mechanical thrombectomy device of claim 15, wherein the strut has flexibility, and
the soldering has a longitudinal length that maintains the flexibility of the strut even when the soldering is attached to the strut coil marker.

17. The mechanical thrombectomy device of claim 15, wherein the adhesive is tapered in a fillet shape.

18. The mechanical thrombectomy device of claim 17, wherein the strut coil marker and a soldering region of the strut have an outer diameter equal to or less than a second threshold value required for delivery characteristics of the thrombectomy device.

19. The mechanical thrombectomy device of claim 8, wherein the strut coil marker is formed of at least one material selected from the group consisting of platinum (Pt), iridium (Ir), stainless steel, gold, and tungsten.
